(19) Europäisches Patentamt
European Patent Office
Office européen des brevets

(11) Numéro de publication: **0 153 212 B1**

# (12) FASCICULE DE BREVET EUROPEEN

(45) Date de publication du fascicule du brevet: **13.07.88**

(51) Int. Cl.⁴: **C 07 F 9/09**, C 07 D 309/30, C 07 D 307/93

(21) Numéro de dépôt: **85400133.6**

(22) Date de dépôt: **28.01.85**

(54) **Nouveaux dérivés de la 4,4-diméthyl tétrahydro-2H-pyran-2-one, leur procédé de préparation et leur application à la synthèse de dérivés cyclopropaniques.**

(30) Priorité: **01.02.84 FR 8401550**

(43) Date de publication de la demande: **28.08.85 Bulletin 85/35**

(45) Mention de la délivrance du brevet: **13.07.88 Bulletin 88/28**

(84) Etats contractants désignés: **AT BE CH DE GB IT LI LU NL SE**

(56) Documents cités:
**EP - A - 0 031 932**
**EP - A - 0 103 749**
**FR - A - 1 588 891**

(73) Titulaire: **ROUSSEL-UCLAF, 35, boulevard des Invalides, F-75007 Paris (FR)**

(72) Inventeur: **Tessier, Jean, 30, rue Jean Moulin, F-94300 Vincennes (FR)**
Inventeur: **Demoute, Jean-Pierre, 249 bis rue de Rosny, F-93100 Montreuil S/Bois (FR)**

(74) Mandataire: **Tonnellier, Marie-José et al, Département des Brevets ROUSSEL UCLAF B.P. no 9, F-93230 Romainville (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

**Description**

L'invention concerne de nouveaux dérivés de la 4,4-diméthyltétrahydro-2H-pyran-2-one, leur procédé de préparation et leur application à la synthèse de dérivés cyclopropaniques.

L'invention a pour objet, sous toutes leurs formes isomères possibles, les composés de formule (I):

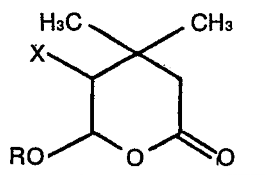

(I)

dans laquelle X représente un groupement nucléofuge choisi dans le groupe constitué par les atomes d'halogène, les radicaux:

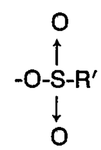

dans lesquels R′ représente un radical alkyle renfermant de 1 à 18 atomes de carbone ou R′ représente un radical aryle renfermant de 6 à 14 atomes de carbone et les radicaux:

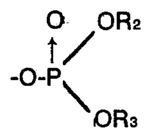

dans lesquels $R_2$ et $R_3$ identiques ou différents, représentent un radical alkyle renfermant de 1 à 18 atomes de carbone ou un radical aryle renfermant de 6 à 14 atomes de carbone et R représente un atome d'hydrogène ou le reste carboné d'un alcool ROH chiral ou non chiral, ainsi que tous les mélanges possibles de ces isomères.

Par atome d'halogène, on entend les atomes de fluor, de chlore, de brome ou d'iode.

Lorsque R′, $R_2$ et $R_3$ représentent un radical alkyle, il s'agit, de préférence, d'un radical méthyle, éthyle, n-propyle, isopropyle, n-butyle ou terbutyle.

Lorsque R′, $R_2$ et $R_3$ représentent un radical aryle, il s'agit, de préférence, d'un radical phényle ou du radical phényle substitué par un ou plusieurs groupements Oalc ou alc, alc représentant un radical alkyle renfermant de 1 à 8 atomes de carbone, ou par un groupement $CF_3$, $OCF_3$ ou $SCF_3$.

R peut représener le reste d'un alcool quelconque. R représente notamment le reste d'un alcool linéaire, ramifié, mono ou polycyclique renfermant jusqu'à 18 atomes de carbone et pouvant contenir un ou plusieurs hétéroatomes. R peut représenter par exemple:

1. un radical alkyle, par exemple, un radical méthyle, éthyle, n-propyle, isopropyle, terbutyle, n-butyle, n-pentyle et n-hexyle;

2. un radical

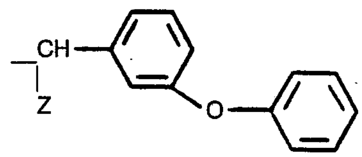

avec Z représentant un atome d'hydrogène, un radical méthyle, éthynyle ou cyano, dans lequel chacun des noyaux phényles peut être substitué par un atome d'halogène;

3. un radical

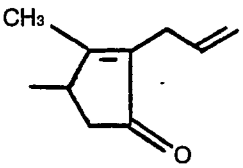

4. un radical

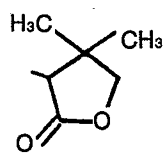

5. un radical

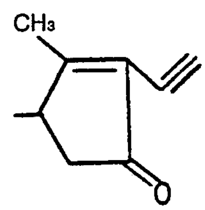

6. un radical

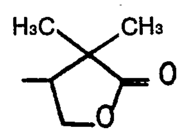

7. un radical

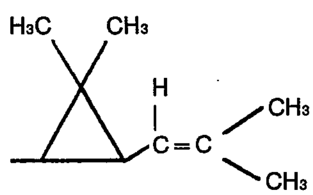

8. un radical

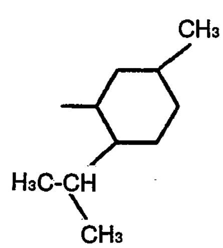

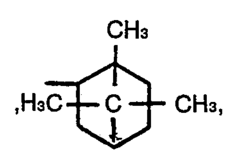

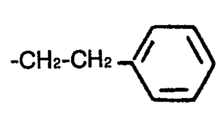

ou

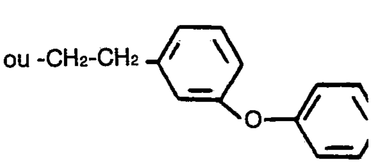

9. un radical

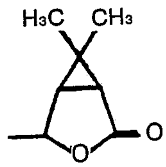

10. un radical

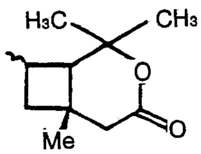

11. un radical cyclopropyle, cyclobutyle, cyclopentyle ou cyclohexyle.

L'invention a notamment pour objet les composés de formule (I) dans laquelle X représente un atome de chlore, de brome ou d'iode ainsi que ceux dans laquelle Y représente un atome d'oxygène.

Comme il a été mentionné ci-dessus, R peut représenter le reste d'alcools très variés; R peut représenter, par exemple, un radical méthyle, éthyle, n-propyle, isopropyle, n-butyle, terbutyle, n-pentyle, n-hexyle, cyclopropyle, cyclobutyle, cyclopentyle ou cyclohexyle.

R peut représenter encore le radical menthyle, isomenthyle, bornyle, isobornyle, phénéthyle ou m-phénoxy-phénéthyle.

L'invention a également tout spécialement pour objet les composés de formule (I) dans lesquels R représente un atome d'hydrogène.

L'invention a bien entendu tout particulièrement pour objet les composés de formule (I) décrits en exemple.

L'invention a également pour objet un procédé de préparation des composés de formule (I), caractérisé en ce que l'on soumet le composé de formule (II):

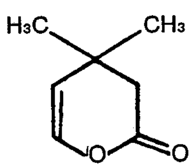

(II)

à l'action simultanée d'un agent permettant d'introduire X, X conservant la même signification que précédemment, et d'un composé de formule ROH, dans laquelle R conserve la même signification que précédemment, pour obtenir le composé de formule (I) correspondant:

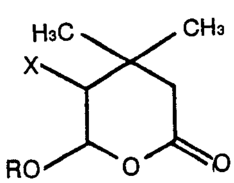

(I)

L'invention a notamment pour objet un procédé de préparation des produits de formule (I') correspondants aux produits de formule (I) dans laquelle X représente un radical $X_1$, $X_1$ représentant un atome de chlore, de brome ou d'iode et Y représente un atome d'oxygène:

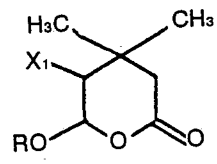

(I')

caractérisé en ce que l'on soumet le composé de formule (II):

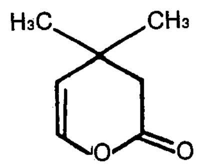

(II)

à l'action simultanée d'un N-halosuccinimide dans lequel halo représente un atome de chlore, de brome ou d'iode et d'un composé ROH dans lequel R conserve la même signification que précédemment, pour obtenir le composé de formule (I') correspondant.

Lorsque $X_1$ représente un atome de chlore et R représente un radical alkyle, on opère de préférence à température ambiante au sein d'un alcool en présence de traces d'acide fort comme l'acide sulfurique.

Lorsque $X_1$ représente un atome de brome ou d'iode et R représente un radical alkyle, on opère de préférence au sein d'un alcool dilué par un tiers solvant à une température comprise entre −50 °C et 20 °C.

L'invention a également pour objet une variante du procédé pour préparer les produits de formule (I') telle que définie précédemment, caractérisée en ce que l'on soumet le composé de formule (II) à l'action d'un agent d'halogénation permettant d'introduire $X_1$, pour obtenir le composé de formule (III):

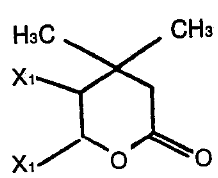

(III)

que l'on soumet ensuite:

– soit à l'action d'un composé de formule $R_1OH$ dans laquelle $R_1$ représente le reste carboné d'un alcool, pour obtenir le composé de formule ($I'_B$) correspondant:

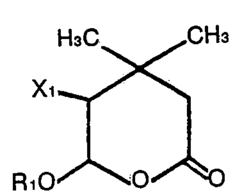

($I'_B$)

dans laquelle $R_1$ et $X_1$ sont définis comme précédemment;

– soit à l'action de l'eau dans des conditions douces pour obtenir les composés (IV) et $(I'_A)$ en équilibre:

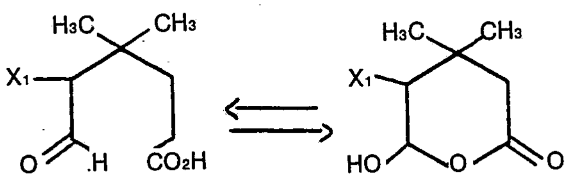

(IV) $(I'_A)$

isole, si désiré, le composé de formule $(I'_A)$, puis soumet, si désiré, soit le composé $(I'_A)$, soit le mélange de composés (IV) et $(I'_A)$ à l'action d'un alcool $R_1OH$ pour obtenir le composé de formule $(I'_B)$:

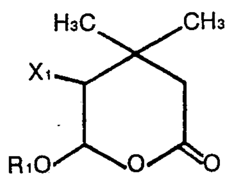

$(I'_B)$

dans laquelle $R_1$ et $X_1$ sont définis comme précédemment;

– soit à l'action de l'eau dans des conditions plus rudes, pour obtenir le composé de formule (V):

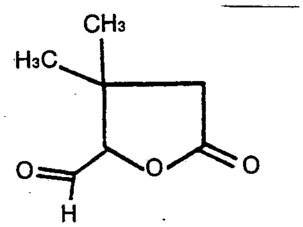

(V)

que l'on soumet à l'action d'un alcool $R_1OH$ pour obtenir le composé de formule (VI):

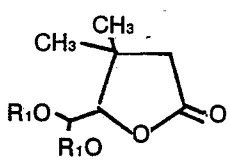

(VI)

que l'on soumet à l'action d'un trihalogénure de bore:

$$(X_1)_3B$$

dans lequel $X_1$ est défini comme précédemment pour obtenir le composé de formule $(I'_B)$:

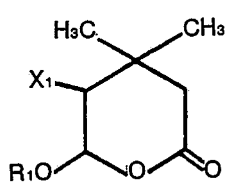

$(I'_B)$

dans laquelle $R_1$ et $X_1$ sont définis comme précédemment.

Dans un mode de réalisation préféré du procédé de l'invention:

– l'agent d'halogénation que l'on fait réagir sur le composé de formule (II) est le chlore, le brome ou l'iode ($Cl_2$, $Br_2$ ou $I_2$), la réaction d'halogénation étant réalisée au sein du tétrachlorure de carbone à une température comprise entre $-50\,°C$ et $+25\,°C$;

– la réaction du composé de formule (III) et de l'eau a lieu à la température ambiante le cas échéant, en présence d'un acide comme l'acide chlorhydrique ou l'acide sulfurique dans des conditions telles que l'hydrolyse soit seulement partielle;

– la réaction d'éthérification du composé de formule $(I'_A)$ par l'alcool $R_1OH$ est réalisée en présence d'un acide fort comme l'acide paratoluène sulfonique;

– la réaction du composé (III) avec l'eau pour obtenir le composé de formule (V) a lieu en milieu acide aqueux dans des conditions telles que l'hydrolyse des deux halogènes $X_1$ soit totale.

Certains produits mis en œuvre lors du procédé de l'invention sont des produits nouveaux, à savoir le produit de formule (V):

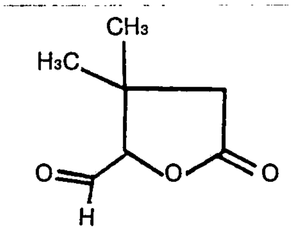

(V)

et les produits de formule (VI):

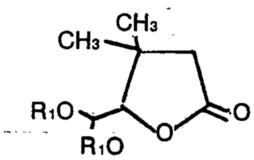

(VI)

dans laquelle $R_1$ représente le reste d'un alcool chiral ou non chiral.

L'invention a donc pour objet le produit de formule (V) et les produits de formule (VI), à titre de produits chimiques nouveaux.

L'invention a, en outre, pour objet l'application des composés de formule (I), caractérisée en ce que l'on soumet un composé de formule (I):

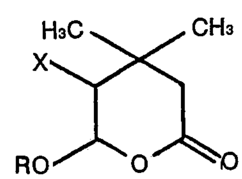

(I)

dans laquelle X et R sont définis comme précédemment, à l'action d'un agent basique pour obtenir le composé de formule (VII):

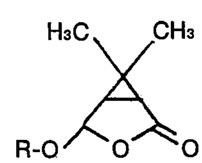

(VII)

dans laquelle R est défini comme précédemment.

Comme agents basiques, on peut utiliser les hydrures métalliques, les alcoolates alcalins ou alcalinoterreux, les dérivés organométalliques, les hydroxydes alcalins ou alcalinoterreux, les carbonates alcalins ou alcalinoterreux et les amines tertiaires.

L'invention a tout spécialement pour objet un procédé de préparation des composés de formule (VII), caractérisé en ce que l'agent basique est un hydroxyde alcalin, comme la soude ou la potasse.

Dans l'application des produits de formule (I), on utilise de préférence l'agent basique selon la méthode dite de catalyse par transfert de phase.

L'invention a tout particulièrement pour objet l'application caractérisée en ce que dans la méthode dite de catalyse par transfert de phase, le solvant est le chlorure de méthylène, le catalyseur est le triéthyl benzyl ammonium, l'agent basique est la soude et l'on opère en présence d'eau.

Les produits de l'invention permettent donc de préparer les produits de formule (VII):

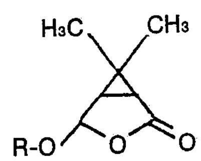

(VII)

dans laquelle R est défini comme précédemment.

Ces produits de formule (VII) sont des produits connus décrits par exemple dans le brevet français 1580474, dans les brevets européens 0023454 et 0024241 et dans «Pesticide Science», volume 11, 2 avril 1980, p. 188–201.

Les produits de formule (VII) peuvent être utilisés dans la préparation d'acides cis cyclopropane carboxyliques diversement substitués, par exemple, les acides cis 2,2-diméthyl 3,3-dihalo vinyliques, qui par estérification, conduisent à des produits insecticides tràs actifs comme la deltaméthrine ou la cis cyperméthrine.

Les exemples suivants illustrent l'invention sans toutefois la limiter.

Exemple 1: (trans) 5-bromo-4,4-diméthyl-6-méthoxy-tétrahydro-2H-pyran-2-one.

On introduit à $-50\ °C \pm 5\ °C$ une solution renfermant 8 g de N-bromo succinimide, 120 $cm^3$ de chlorure de méthylène et 30 $cm^3$ de méthanol dans une solution renfermant 5 g de 3,4-dihydro 4,4-diméthyl α-pyrone et 80 $cm^3$ de chlorure de méthylène. On maintient le mélange réactionnel sous agitation à $-50\ °C \pm 5\ °C$ pendant 2 heures et demie environ. On laisse revenir à la température ambiante, lave à l'eau, à l'aide d'une solution de bicarbonate de soude, sèche et amène à sec. On obtient 10,5 g de produit que l'on chromatographie sur silice en éluant par le mélange hexane-éther isopropylique (1/1). On obtient 7,2 g de produit recherché fondant au dessous de 50 °C.

Exemple 2: (trans) 5-bromo-4,4-diméthyl-6-(1,1-diméthyl éthoxy) tétrahydro-2H-pyran-2-one.

On ajoute en 45 minutes à $-30$ °C une solution renfermant 8 g de N-bromo succinimide, 140 $cm^3$ de chlorure de méthylène et 30 $cm^3$ de terbutanol dans une solution renfermant 5 g de 3,4-dihydro 4,4-diméthyl α-pyrone dans 80 $cm^3$ de chlorure de méthylène. On laisse le mélange réactionnel revenir à la température ambiante, l'agite pendant 45 h, le lave à l'eau puis à l'aide d'une solution saturée de chlorure de sodium, le sèche et l'amène à sec. On obtient 12 g de produit que l'on chromatographie sur silice en éluant par le mélange hexane-éther isopropylique. On obtient 2,86 g de produit recherché fondant à 104 °C.

Exemple 3: 5R /5α, 6β(1R,2S,5R)/ 5-bromo-4,4-diméthyl-6-/5-méthyl-2-(1-méthyl éthyl) 1-cyclohexyloxy/ tétrahydro 2H-pyran-2-one (isomère A) et composé 5S correspondant (isomère B).

On ajoute 3,5 g de N-bromo succinimide dans une solution renfermant 2,5 g de 3,4-dihydro 4,4-diméthyl α-pyrone, 3,3 g de 1-menthol et 20 $cm^3$ de tétrachlorure de carbone. On maintient le mélange réactionnel sous agitation pendant 2 jours à la température ambiante. On dilue à l'éther isopropylique, essore l'insoluble et évapore le filtrat à sec. On chromatographie sur silice le produit obtenu en éluant par le mélange hexane-acétate d'éthyle (9-1). On isole ainsi 1,1 g d'isomère A fondant à 68 °C $/\alpha/_D = -100°$ (c = 1% $CHCl_3$) et 0,5 g d'isomère B fondant à 77 °C. $/\alpha/_D = -1°,5$ (c = 2% $CHCl_3$).

Exemple 4: trans 5-chloro 4,4-diméthyl-6-(1-méthyl éthoxy)-tétrahydro-2H-pyran-2-one.

On ajoute à 0 °C 3 g de N-chloro succinimide et 2 gouttes d'une solution concentrée d'acide sulfurique dans une solution renfermant 2,5 g de 3,4-dihydro 4,4-diméthyl pyrone dans 20 $cm^3$ d'isopropanol. On maintient le mélange réactionnel sous agitation pendant 115 heures à la température ambiante, dilue avec du chlorure de méthylène, lave à l'aide d'une solution saturée de chlorure de sodium et avec une solution de carbonate de soude à 10%, sèche et amène à sec. On obtient 5,8 g de produit que l'on chromatographie sur silice en éluant par le mélange hexane-éther isopropylique (7/3). On obtient 1,47 g du produit recherché.

Exemple 5: trans 4,4-diméthyl 5-iodo-6-(1-méthyl éthoxy)-tétrahydro-2H-pyran-2-one.

On ajoute à 0 °C 10 g de N-iodo succinimide et 2 gouttes d'une solution d'acide sulfurique concentrée dans une solution renfermant 5 g de 3,4-dihydro 4,4-diméthyl pyrone et 40 $cm^3$ d'isopropanol. On laisse revenir à la température ambiante, et maintient sous agitation pendant 5 heures 30. On dilue au chlorure de méthylène, lave avec une solution aqueuse de chlorure de sodium, puis avec une solution à 10% de carbonate de sodium. On sèche et concentre à sec. On obtient 13 g de produit que l'on chromatographie sur silice en éluant par le mélange hexane-éther isopropylique 7/3. On obtient 7,64 g de produit recherché fondant à 60 °C.

Exemple 6: (trans) 5-chloro-4,4-diméthyl 6-hydroxy tétrahydro-2H-pyran-2-one.

Stade a: 5,6-dichloro-4,4-diméthyl-tétrahydro-2H-pyran-2-one.

On fait barboter à 0 °C un courant de chlore pendant 10 minutes dans une solution renfermant 5 g de 3,4-dihydro 4,4-diméthyl pyrone et 50 $cm^3$ de tétrachlorure de carbone. On distille à sec et obtient 7,78 g de produit recherché.

Stade B: (trans) 5-chloro-4,4-diméthyl 6-hydroxy-tétrahydro-2H-pyran-2-one.

On agite 5 heures 7,5 g du dérivé dichloré décrit au stade A dans un mélange renfermant 50 $cm^3$ d'acétone et 25 $cm^3$ d'acide chlorhydrique (N). On extrait au chlorure de méthylène, purifie le mélange obtenu par chroamtographie sur silice (éluant: hexane-acétate d'éthyle 1-1). On obtient ainsi 4,4 g du produit recherché sous forme de résine.

Exemple 7: (trans) 5-chloro-4,4-diméthyl-6-(1-méthyl éthoxy)- tétrahydro-2H-pyran-2-one.

On ajoute 0,95 $cm^3$ d'isopropanol et 40 mg d'acide paratoluène sulfonique monohydraté dans une solution renfermant 2 g de produit préparé à l'exemple 6B et 30 $cm^3$ de chlorure de méthylène. On porte le mélange réactionnel au reflux à 25 °C/ 28 °C sous pression réduite. On maintient le reflux pendant 15 heures. On maintient encore sous agitation à la température ambiante pendant 8 heures et l'on ajoute 10 $cm^3$ de chlorure de méthylène, 0,95 $cm^3$ d'isopropanol et 5 mg d'acide paratoluène sulfonique monohydraté. On porte à nouveau au reflux pendant 7 heures, dilue au chlorure de méthylène, lave à l'aide d'une solution saturée de chlorure de sodium et avec une solution de carbonate de soude. On sèche sur sulfate de soude et amène à sec. On obtient 1,9 g de produit que l'on chromatographie sur silice en éluant par le mélange hexane-éther isopropylique (7/3). On obtient 1,1 g de produit recherché.

Exemple 8: 5R /5α,6β(1R,2S,5R)/ 5-chloro-4,4-diméthyl-6-/5-méthyl-2-(1-méthyl éthyl) 1-cyclohexyloxy/ tétrahydro-2H-pyran-2-one (isomère A) et composé 5S correspondant (isomère B).

On ajoute 1,9 g de 1-menthol et 40 mg d'acide paratoluène sulfonique dans une solution renfermant 2 g du produit préparé à l'exemple 6B et 30 $cm^3$ de chlorure de méthylène. On porte le mélange réactionnel au reflux sous pression réduite à 25 °C/28 °C et agite pendant 45 heures. On dilue au chlorure de méthylène, lave avec une solution aqueuse saturée de chlorure de sodium puis avec une solution de carbonate de soude, sèche et distille à sec. On obtient 3,5 g de résine que l'on chromatographie sur silice en éluant par le mélange hexane-éther isopropylique (7/3). On obtient ainsi 0,94 g de produit recherché fondant à 80 °C (isomère A) et 0,5 g de produit recherché (isomère B) fondant à 70 °C.

Exemple 9: 5R/ 5α,6β(1R,2S,5R)/ 5-bromo-4,4-diméthyl-6-/5-méthyl-2-(1-méthyl éthyl) 1-cyclohexyloxy/tétrahydro 2H-pyran-2-one (isomère A) et composé 5S correspondant (isomère B).

Stade A: trans 5,6-dibromo-4,4-diméthyl-tétrahydro-2H-pyran-2-one.

On ajoute goutte à goutte à 0 °C–5 °C une solution renfermant 1,6 $cm^3$ de brome dans 5 $cm^3$ de tétrachlorure de carbone, dans une solution renfermant 3,78 g de 3,4-dihydro-4,4-diméthyl α-pyrone et 10 $cm^3$ de tétrachlorure de carbone. On évapore à sec sans chauffer et cristallise dans l'éther isopropylique pour obtenir 5,5 g de produit recherché fondant à 90/92 °C qui sera conservé sous argon à 5 °C.

Stade B: 5R/ 5α,6β(1R,2S,5R)/ 5-bromo-4,4-diméthyl-6-/5-méthyl-2-(1-méthyl éthyl) 1-cyclohexyloxy/tétrahydro 2H-pyran-2-one.

On agite pendant 29 heures à la température ambiante 0,82 g de 1-menthol, 1 g du produit préparé au stade A, 10 mg d'acide paratoluène sulfonique monohydraté et 10 $cm^3$ de chlorure de méthylène. On lave avec une solution de carbonate de soude à 10% et extrait au chlorure de méthylène. On lave avec une solution saturée de chlorure de sodium et sèche les phases chlorométhyléniques, les sèche, les concentre et obtient une huile que l'on chromatographie sur silice en éluant par le mélange hexane-éther isopropylique (8/2). On obtient 0,24 g de produit recherché (isomère A) fondant à 68 °C et 0,15 g de produit recherché (isomère B) fodnant à 80 °C.

Exemple 10: (trans) 5-bromo-4,4-diméthyl-6-hydroxy tétrahydro-2H-pyran-2-one.

On place 468 mg de produit obtenu au stade A de l'exemple 9 dans un récipient ouvert et le maintient pendant 4 heures à 25° au contact de l'air ambiant humide. On reprend le produit obtenu par du chlorure de méthylène, le sèche et évapore à sec sous pression réduite. On obtient 310 mg de produit recherché fondant à une température inférieure à 50 °C.

Exemple 11: 5R /5α,6β(1R,2S,5R)/ 5-bromo-4,4-diméthyl-6-/5-méthyl-2-(1-méthyl éthyl)1-cyclohexyloxy/ tétrahydro 2H-pyran-2-one.

On introduit 1 g de produit préparé à l'exemple précédent dans une solution renfermant 30 $cm^3$ de chlorure de méthylène, 0,75 g de 1-menthol et 0,01 g d'acide paratoluène sulfonique monohydraté. On maintient pendant 48 heures le mélange réactionnel au reflux sous une pression de 190 mm de mercure (Eb 24 °C). On lave le milieu réactionnel à l'aide d'une solution saturée de chlorure de sodium et extrait au chlorure de méthylène. On rassemble les phases chlorométhyléniques, les sèche et les concentre. On obtient 1,5 g de produit que l'on chromatographie sur silice en éluant par le mélange hexane-éther isopropylique (75/25). On obtient ainsi 0,46 g de produit recherché fondant à 68 °C.

Exemple 12: /5R /5α,6β(1R,2S,5B)/5-bromo 4,4-diméthyl 6-/5-méthyl-2-(1-méthyl éthyl) 1-cyclohexyloxy/ tétrahydro 2H-pyran-2-one.

Stade A: 3,3-diméthyl 4-formyl 4-butanolide.

On agite à la température ambiane pendant 5 jours 2,96 g de trans 5,6-dibromo-4,4-diméthyl tétrahydro-2H-pyran-2-one préparé selon l'exemple 9A 25 $cm^3$ d'acétone, 10 $cm^3$ d'eau et 1 $cm^3$ d'acide chlorhydrique 0,1N. On extrait au chlorure de méthylène, lave la phase organique à l'eau, la sèche et la concentre. On obtient 1,47 g d'une huile brute que l'on chromatographie sur silice en éluant par le mélange hexane-acétate d'éthyle (3/7). On obtient 1,3 g d'une huile renfermant le produit recherché.

Stade B: (4R ou S) 3,3-diméthyl 4-/1,1-di/(1R,2S,5R) 2-isopropyl 5-méthyl cyclohexyl-oxy// méthyl 4-butanolide.

On maintient au reflux pendant 48 heures (température voisine de 45 °C) 4 g de 3,3-diméthyl 4-formyl 4-butanolide, 8,8 g de 1-menthol, 50 cm³ de chlorure de méthylène et 0,2 g d'acide paratoluène sulfonique. On refroidit le milieu réactionnel et le lave avec une solution aqueuse de bicarbonate de soude puis une solution saturée de chlorure de sodium. On rassemble les phases chlorométhyléniques, les sèche et les concentre sous pression réduite. On obtient 11,77 g d'une huile que l'on chromatographie sur silice en éluant par le mélange hexane-éther isopropylique (8/2). On récupère ainsi 3,2 g d'isomère A, F = 65 °C et 3,29 g d'isomère B, F = 118 °C.

Stade C: /5R /5α,6β(1R,2S,5R)/ 5-bromo 4,4-diméthyl 6-/5-méthyl 2-(1-méthyl éthyl) 1-cyclohexyl-oxy/ tétrahydro-2H-pyran-2-one.

On agite à la température ambiante pendant 20 heures 1,31 g de (4-S ou R) 3,3-diméthyl 4-/1,1-di/(1R,2S,5R) 2-isopropyl 5-méthyl cyclohexyl-oxy// méthyl-4-butanolide (isomère B), 13 cm³ de chlorure de méthylène et 3,5 cm³ d'une solution molaire de tribromure de bore dans le chlorure de méthylène. On verse le mélange réactionnel dans de la glace en présence d'une solution de carbonate de sodium à 10%. On extrait au chlorure de méthylène, lave, sèche les phases chlorométhyléniques, les concentre à sec. On obtient 0,8 g d'une huile brute que l'on chromatographie sur silice (en éluant par le mélange hexane-éther isopropylique 75/25). On obtient ainsi 0,17 g de produit recherché, F = 68 °C.

Exemple 13: (trans) 5-bromo-4,4-diméthyl-6-hydroxy tétrahydro-2H-pyran-2-one.

On dissout 10 g de 3,4-dihydro 4,4-diméthyl-α-pyrone dans 100 cm³ de diméthyl sulfoxyde. On ajoute 2,9 cm³ d'eau. On refroidit aux alentours de 10 °C. On ajoute 17,8 g de N-bromo succinimide. On agite la solution obtenue au bain de glace pendant 5 minutes, puis on laisse revenir à la température ambiante en agitant pendant 1 heure. On verse sur une solution de chlorure de sodium, extrait à l'éther, sèche et distille à sec. On obtient 17,5 g de produit que l'on recristallise dans l'éther isopropylique. On obtient le produit recherché sous forme de cristaux blancs fondant à 55 °C.

L'analyse spectrale RMN montre que le produit recherché et sa forme ouverte sont en équilibre:

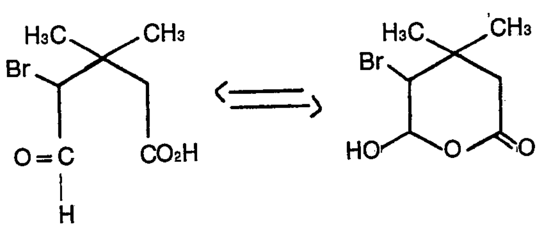

Application 1: 6,6-diméthyl-4-méthoxy-3-oxabicyclo /3-1-0/ hexan-2-one.

On verse à 0 °C 15 cm³ de solution aqueuse de soude à 50% puis 0,28 g de chlorure de triéthylbenzylammonium dans une solution renfermant 2,78 g de produit préparé à l'exemple 1 et 30 cm³ de chlorure de méthylène. On laisse revenir à la température ambiante. On porte ensuite à 45 °C–50 °C le mélange réactionnel et agite pendant 2 h 30. On laisse revenir à la température ambiante, dilue au chlorure de méthylène, décante, lave à l'eau, sèche et amène à sec. On obtient 1,22 g de produit recherché.

Application 2: 6,6-diméthyl-4-(1,1-diméthyléthoxy) 3-oxabicyclo /3-1-0/ hexan-2-one.

On ajoute à 0–5 °C une solution renfermant 5 cm³ d'une solution de soude à 50% et 50 mg de chlorure de triéthylbenzylammonium, dans une solution renfermant 1,4 g du produit préparé à l'exemple 2 et 7 cm³ de chlorure de méthylène. On laisse revenir à la température ambiante et agite pendant 24 heures. On dilue avec le minimum d'eau, sature par du chlorure de sodium, décante et extrait au chlorure de méthylène. On réunit les phases organiques, lave avec une solution saturée de chlorure de sodium, sèche et amène à sec. On obtient 610 mg d'un produit que l'on recristallise dans l'isopropanol et obtient 370 mg de produit fondant à 115 °C.

Application 3: /1R /1α,4α(1R,2S,5R) 5// 6,6-diméthyl-4-//5-méthyl-2-(1-méthyl éthyl) cyclohexyl// oxy 3-oxa bicyclo /3-1-0/ hexan-2-one.

On ajoute 2 cm³ d'une solution de soude à 50% et 20 mg de chlorure de triéthyl benzyl ammonium dans une solution renfermant 360 mg de produit préparé à l'exemple 3 dans 5 cm³ de chlorure de méthylène. On laisse le mélange réactionnel sous agitation pendant 24 heures, ajoute 15 mg de chlorure de triéthyl benzyl ammonium. On maintient à nouveau le mélange réactionnel sous agitation pendant 7 heures. On dilue à l'eau, extrait au chlorure de méthylène. On chromatographie le produit obtenu sur silice en éluant avec du chlorure de méthylène. On obtient 275 mg de produit recherché F = 82 °C. $\alpha_D = -197° + 4°$ (c = 0,5% $CHCl_3$).

Application 4: 6,6-diméthyl-4-(1-méthyl éthoxy) 3-oxa bicyclo /3-1-0/ hexan-2-one.

On ajoute à 0 °C 2 cm³ d'une solution aqueuse de soude à 50% et 30 mg de chlorure de triéthyl benzyl ammonium dans une solution renfermant 660 mg de produit préparé à l'exemple 4 et 6 cm³ de chlorure de méthylène. On maintient le mélange réactionnel sous agitation pendant 5 heures à la température ambiante. On dilue au chlorure de méthylène, décante, dilue la phase alcaline à l'eau, extrait au chlorure de méthylène, réunit les phases organiques, lave avec une solution aqueuse saturée en chlorure de sodium, sèche et amène à sec. On obtient 440 mg de produit recherché.

Application 5: 6,6-diméthyl-4-(1-méthyl éthoxy) 3-oxa bicyclo /3-1-0/ hexan-2-one.

On ajoute à 0 °C 6 cm³ d'une solution aqueuse de soude à 50% et 60 mg de chlorure de triéthyl benzyl ammonium dans une solution renfermant 1,56 g de produit de l'exemple 5 et 15 cm³ de chlorure de

méthylène. On maintient le mélange réactionnel sous agitation pendant 20 heures à la température ambiante. On dilue avec du chlorure de méthylène, décante, dilue la phase alcaline à l'eau, l'extrait au chlorure de méthylène. On réunit les phases organiques, les lave à l'aide d'une solution saturée de chlorure de sodium, les sèche et les concentre à sec. On obtient 0,52 g du produit recherché.

Application 6: /1R /1α,4α(1R,2S,5R) 5/ 6,6-diméthyl-4-//5-méthyl-2-(1-méthyl éthyl) cyclohexyl/ oxy/ 3-oxa bicyclo /3-1-0/ hexan-2-one.

On ajoute à 0 °C 3 $cm^3$ d'une solution aqueuse de soude à 50% et 30 mg de chlorure de triéthyl benzyl ammonium dans une solution renfermant 732 mg du produit préparé à l'exemple 8 et 8 $cm^3$ de chlorure de méthylène. On agite à la température ambiante pendant 5 heures 30. On décante, dilue a phase aqueuse à l'eau, extrait au chlorure de méthylène, réunit les phases organiques, lave, sèche et amène à sec. On obtient 0,59 g de produit que l'on chromatographie sur silice en éluant par le dichloréthane. On obtient ainsi 509 mg du produit recherché fondant à 82 °C.

**Revendications**

1. Sous toutes leurs formes isomères possibles, les composés de formule (I):

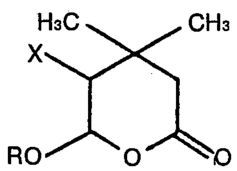

(I)

dans laquelle X représente un groupement nucléofuge choisi dans le groupe constitué par les atomes d'halogène, les radicaux:

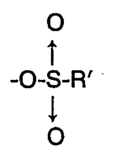

dans lesquels R' représente un radical alkyle renfermant de 1 à 18 atomes de carbone ou R' représente un radical aryle renfermant de 6 à 14 atomes de carbone et les radicaux:

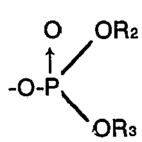

dans lesquels $R_2$ et $R_3$ identiques ou différents, représentent un radical alkyle renfermant de 1 à 18 atomes de carbone ou un radical aryle renfermant de 6 à 14 atomes de carbone et R représente un atome d'hydrogène ou le reste carboné d'un alcool ROH chiral ou non chiral, ainsi que tous les mélanges possibles de ces isomères.

2. Les composés de formule (I) tels que définis à la revendication 1 caractérisés en ce que X représente un atome de chlore, de brome ou d'iode.

3. Les composés de formule (I) tels que définis à l'une quelconque des revendications 1 et 2 caractérisés en ce que R représente un radical méthyl, éthyle, n-propyle, isopropyle, n-butyle, terbutyle, n-pentyle, n-hexyle, cyclopropyle, cyclobutyle, cyclopentyle ou cyclohexyle.

4. Les composés de formule (I) tels que définis à l'une quelconque des revendications 1 et 2 caractérisés en ce que R représente le radical menthyle, isomenthyle, bornyle, isobornyle, phénéthyle ou m-phénoxy phénéthyle.

5. Les composés de formule (I) tels que définis à l'une quelconque des revendications 1 et 2 caractérisés en ce que R représente un atome d'hydrogène.

6. Procédé de préparation des composés de formule (I) tels que définis à l'une quelconque des revendications 1 à 5, caractérisé en ce que l'on soumet le composé de formule (II):

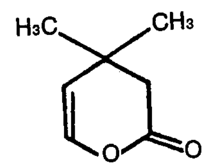

(II)

à l'action simultanée d'un agent permettant d'introduire X, X conservant la même signification que dans la revendication 1, et d'un composé de formule ROH dans laquelle R conserve la même signification que dans la revendication 1, pour obtenir le composé de formule (I) correspondant:

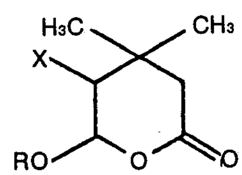

(I)

7. Procédé de préparation selon la revendication 6 des produits de formule (I') correspondants aux produits de formule (I) dans laquelle X représente un radical $X_1$, $X_1$ représentant un atome de chlore, de brome ou d'iode

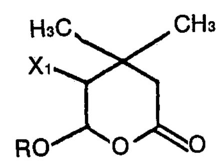

(I')

caractérisé en ce que l'on soumet le composé de formule (II):

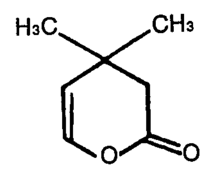

(II)

à l'action simultanée d'un N-halosuccinimide dans lequel halo représente un atome de chlore, de brome ou d'iode et d'un composé ROH dans lequel R conserve la même signification que dans la revendication 1, pour obtenir le composé de formule (I') correspondant.

8. Variante du procédé de la revendication 6 pour préparer les composés de formule (I′) tels que définis à la revendication 7, caractérisée en ce que l'on soumet le composé de formule (II) à l'action d'un agent d'halogénation permettant d'introduire $X_1$, pour obtenir le composé de formule (III):

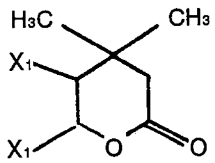

(III)

que l'on soumet ensuite:

– soit à l'action d'un composé de formule $R_1OH$ dans laquelle $R_1$ représente le reste carboné d'un alcool, pour obtenir le composé de formule (I′$_B$) correspondant:

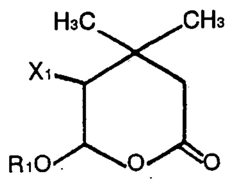

. (I′$_B$)

dans laquelle $R_1$ et $X_1$ sont définis comme précédemment,

– soit à l'action e l'eau dans des conditions douces pour obtenir les composés (IV) et (I′$_A$) en équilibre:

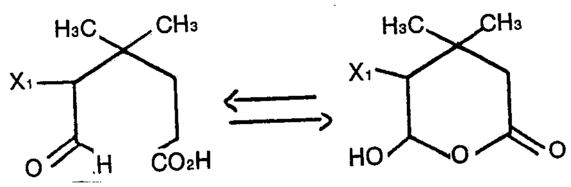

(IV) (I′$_A$)

isole, si désiré, le composé de formule (I′$_A$), puis soumet, si désiré, soit le composé (I′$_A$), soit le mélange de composés (IV) et (I′$_A$) à l'action d'un alcool $R_1OH$ pour obtenir le composé de formule (I′$_B$):

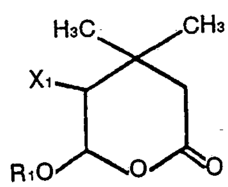

(I′$_B$)

dans laquelle $R_1$ et $X_1$ sont définis comme précédemment,

– soit à l'action de l'eau dans des conditions plus rudes, pour obtenir le composé de formule (V):

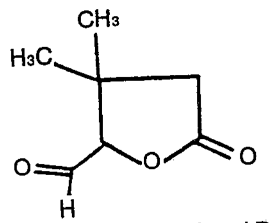

(V)

que l'on soumet à l'action d'un alcool $R_1OH$ pour obtenir le composé de formule (VI):

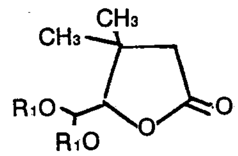

(VI)

que l'on soumet à l'action d'un trihalogénure de bore:

$(X_1)_3B$

dans lequel $X_1$ est défini comme précédemment pour obtenir le composé de formule (I′$_B$):

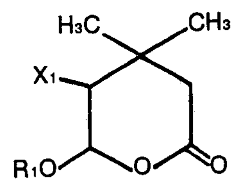

. (I′$_B$)

dans laquelle $R_1$ et $X_1$ sont définis comme précédemment.

9. A titre de produits industriels nouveaux, les composés de formule (V) et (VI):

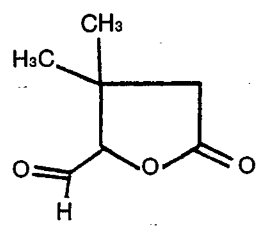

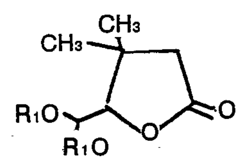

(V) (VI)

dans laquelle $R_1$ représente le reste d'un alcool.

10. Application des composés de formule (I) tels que définis à l'une quelconque des revendications 1 à 5, caractérisée en ce que l'on soumet un composé de formule (I):

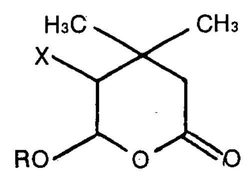

(I)

dans laquelle X et R sont définis comme dans la revendication 1, à l'action d'un agent basique pour obtenir le composé de formule (VII):

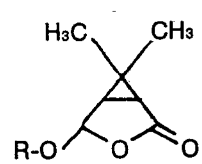

(VII)

dans laquelle R est défini comme dans la revendication 1.

11. Application selon la revendication 10, caractérisé en ce que l'agent basique est un hydroxyde alcalin.

12. Application selon la revendication 10 ou 11,

caractérisée en ce que l'agent basique est utilisé selon la méthode dite de catalyse par transfert de phase.

13. Application selon la revendication 12, caractérisée en ce que dans la méthode dite de catalyse par transfert de phase, le solvant est le chlorure de méthylène, le catalyseur est le triéthyl benzyl ammonium, l'agent basique est la soude et l'on opère en présence d'eau.

**Patentansprüche**

1. In sämtlichen ihrer möglichen isomeren Formen die Verbindungen der Formel (I)

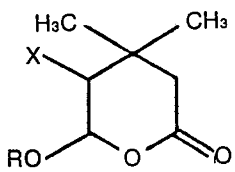

(I)

worin X eine vom Kern abspaltbare Gruppe, ausgewählt unter den Halogenatomen, den Resten

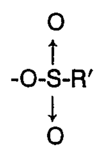

worin R' für einen Alkylrest mit 1 bis 18 Kohlenstoffatomen steht oder R' einen Arylrest mit 6 bis 14 Kohlenstoffatomen bedeutet, und den Resten

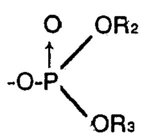

worin $R_2$ und $R_3$, die gleich oder voneinander verschieden sein können, für einen Alkylrest mit 1 bis 18 Kohlenstoffatomen oder einen Arylrest mit 6 bis 14 Kohlenstoffatomen stehen, bedeutet und R ein Wasserstoffatom oder den Kohlenstoffrest eines chiralen oder achiralen Alkohols ROH darstellt, sowie sämtliche möglichen Gemische dieser Isomeren.

2. Verbindungen der Formel (I) gemäß Anspruch 1, dadurch gekennzeichnet, daß X ein Chlor-, Brom- oder Jodatom bedeutet.

3. Verbindungen der Formel (I) gemäß einem der Ansprüche 1 und 2, dadurch gekennzeichnet, daß R für einen Methyl-, Ethyl-, n-Propyl-, Isopropyl-, n-Butyl-, tert.-Butyl-, n-Pentyl-, n-Hexyl-, Cyclopropyl-, Cyclobutyl-, Cyclopentyl- oder Cyclohexylrest steht.

4. Verbindungen der Formel (I) gemäß einem der Ansprüche 1 und 2, ddurch gekennzeichnet, daß R für einen Menthyl-, Isomenthyl-, Bornyl-, Isobornyl-, Phenethyl- oder m-Phenoxyphenethylrest steht.

5. Verbindungen der Formel (I) gemäß einem der Ansprüche 1 und 2, dadurch gekennzeichnet, daß R ein Wasserstoffatom bedeutet.

6. Verfahren zur Herstellung von Verbindungen der Formel (I) gemäß einem der Ansprüche 1 bis 5, dadurch gekennzeichnet, daß man die Verbindung der Formel (II)

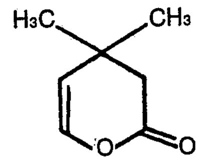

(II)

der gleichzeitigen Einwirkung eines Mittels, das es ermöglicht, X einzuführen, wobei X die in Anspruch 1 angegebene Bedeutung besitzt, und einer Verbindung der Formel ROH, in der R die in Anspruch 1 angegebene Bedeutung besitzt, unterzieht, um die entsprechende Verbindung der Formel (I)

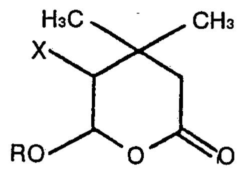

(I)

zu erhalten.

7. Verfahren gemäß Anspruch 6 zur Herstellung der Produkte der Formel (I') entsprechend den Produkten der Formel (I), worin X einen Rest $X_1$ darstellt, wobei $X_1$ ein Chlor-, Brom- oder Jodatom bedeutet,

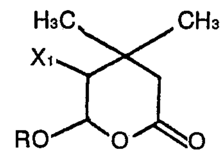

(I')

dadurch gekennzeichnet, daß man die Verbindung der Formel (II)

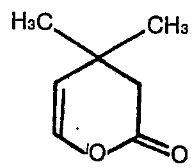

(II)

der gleichzeitigen Einwirkung eines N-Halosuccinimids, in dem Halo für ein Chlor-, Brom- oder Jodatom steht, und einer Verbindung ROH, in der R die in Anspruch 1 angegebene Bedeutung besitzt, unterzieht, um die entsprechende Verbindung der Formel (I') zu erhalten.

8. Abänderung des Verfahrens gemäß Anspruch 6 zur Herstellung von Verbindungen der Formel (I'), wie in Anspruch 7 definiert, dadurch gekennzeichnet, daß man die Verbindung der Formel (II) der Einwirkung eines Halogenierungsmittels unterzieht, das es ermöglicht, $X_1$ einzuführen, um die Verbindung der Formel (III)

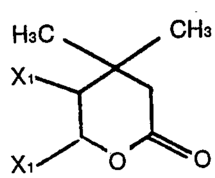

(III)

zu erhalten, die man hierauf

entweder der Einwirkung einer Verbindung der Formel $R_1OH$ unterzieht, worin $R_1$ den Kohlenstoffrest eines Alkohols bedeutet, um die entsprechende Verbindung der Formel ($I'_B$)

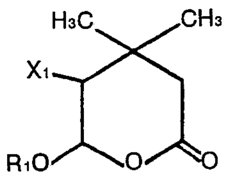

($I'_B$)

zu erhalten, worin $R_1$ und $X_1$ wie vorstehend definiert sind;

oder der Einwirkung von Wasser unter milden Bedingungen unterzieht, um die Verbindungen (IV) und ($I'_A$) im Gleichgewicht

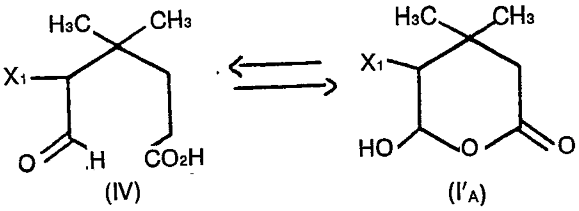

(IV) ($I'_A$)

zu erhalten, gewünschtenfalls die Verbindung der Formel ($I'_A$) isoliert, hiernach gewünschtenfalls entweder die Verbindung der Formel ($I'_A$) oder das Gemisch der Verbindungen (IV) und ($I'_A$) der Einwirkung eines Alkohols $R_1OH$ unterzieht, um die Verbindung der Formel ($I'_B$)

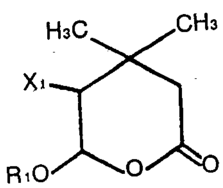

($I'_B$)

zu erhalten, worin $R_1$ und $X_1$ wie vorstehend definiert sind;

oder der Einwirkung von Wasser unter schärferen Bedingungen unterzieht, um die Verbindung der Formel (V)

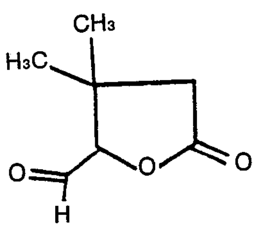

(V)

zu erhalten, die man der Einwirkung eines Alkohols $R_1OH$ unterzieht, um die Verbindung der Formel (VI)

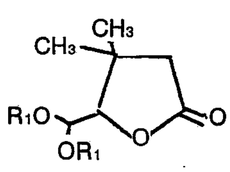

(VI)

zu erhalten, welche man der Einwirkung eines Bortrihalogenids

$(X_1)_3B$

aussetzt, worin $X_1$ die vorstehende Bedeutung besitzt, um die Verbindung der Formel ($I'_B$)

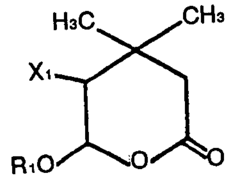

($I'_B$)

zu erhalten, in der $R_1$ und $X_1$ wie vorstehend definiert sind.

9. Als neue, industrielle Produkte die Verbindungen der Formeln (V) und (VI)

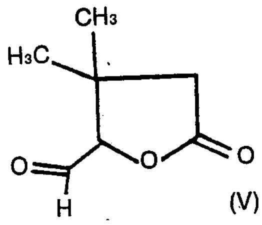

(V)

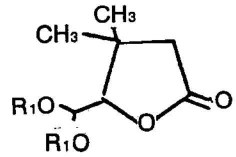

(VI)

worin $R_1$ den Rest eines Alkohols darstellt.

10. Verwendung der Verbindungen der Formel (I) gemäß einem der Ansprüche 1 bis 5, dadurch gekennzeichnet, daß man eine Verbindung der Formel (I)

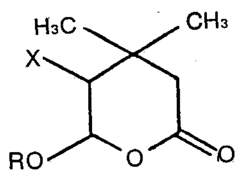

(I)

worin X und R wie in Anspruch 1 definiert sind, der Einwirkung eines basischen Mittels unterzieht, um die Verbindung der Formel (VII)

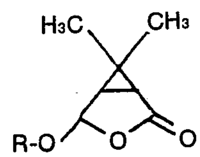

(VII)

zu erhalten, worin R wie in Anspruch 1 definiert ist.

11. Verwendung gemäß Anspruch 10, dadurch gekennzeichnet, daß das basische Mittel ein Alkalihydroxid ist.

12. Verwendung gemäß Anspruch 10 oder 11, dadurch gekennzeichnet, daß das basische Mittel nach der sog. Phasentransferkatalyse-Methode verwendet wird.

13. Verwendung gemäß Anspruch 12, dadurch gekennzeichnet, daß bei der sog. Phasentransferkatalyse-Methode das Lösungsmittel Methylenchlorid ist, der Katalysator Triethylbenzylammonium ist, das basische Mittel Natronlauge ist und man in Anwesenheit von Wasser arbeitet.

**Claims**

1. In all their possible isomeric forms, the compounds with the formula (I):

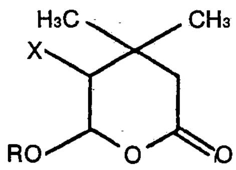

(I)

in which X represents a nucleofuge group chosen from the group constituted by halogen atoms, the radicals:

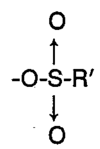

in which R′ represents an alkyl radical containing from 1 to 18 carbon atoms or R′ represents an aryl radical containing from 6 to 14 carbon atoms and the radicals:

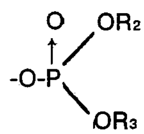

in which $R_2$ and $R_3$, identical or different, represent an alkyl radical containing from 1 to 18 carbon atoms or an aryl radical containing from 6 to 14 carbon atoms and R represents a hydrogen atom or the carbonated residue of a chiral or non-chiral alcohol ROH, as well as all the possible mixtures of these isomers.

2. Compounds with the formula (I) as defined in claim 1 characterized in that X represents a chlorine, bromine or iodine atom.

3. Compounds with the formula (I) as defined in one of claims 1 and 2 characterized in that R represents one of the following radicals: methyl, ethyl, n-propyl, isopropyl, n-butyl, tertbutyl, n-pentyl, n-hexyl, cyclopropyl, cyclobutyl, cyclopentyl or cyclo hexyl.

4. Compounds with the formula (I) as defined in one of claims 1 and 2 characterized in that R represents one of the following radicals: menthyl, isomenthyl, bornyl, isobornyl, phenethyl or m-phenoxyphenethyl.

5. Compounds with the formula (I) as defined in one of claims 1 and 2 characterized in that R represents a hydrogen atom.

6. Preparation process for compounds with the formula (I) as defined in any one of claims 1 to 5, characterized in that a compound with the formula (II):

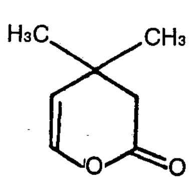

(II)

is submitted to the action of an agent enabling X to be introduced, X keeping the same significance as in claim 1, and of a compound with the formula ROH in which R keeps the same significance as in claim 1, in order to obtain the corresponding compound with the formula (I):

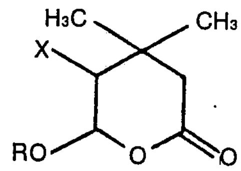

(I)

7. Preparation process according to claim 6 for products with the formula (I′) corresponding to products with the formula (I) in which X represents a radical $X_1$, $X_1$ representing a chlorine, bromine or iodine atom

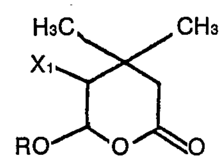

(I′)

characterized in that the compound with the formula (II):

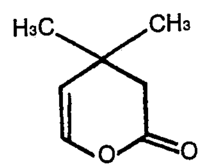

(II)

is submitted to the simultaneous action of an N-halosuccinimide in which halo represents a chlorine, bromine or iodine atom and of a compound ROH in which R keeps the same significance as in claim 1, in order to obtain the corresponding compound with the formula (I′).

8. Variant of the process of claim 6 for preparing compounds with the formula (I′) as defined in claim 7, characterized in that the compound with the formula (II) is submitted to the action of a halogenation agent enabling $X_1$ to be introduced, in order to obtain the compound with the formula (III):

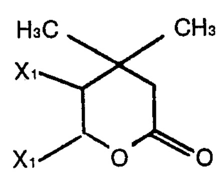

(III)

which is then submitted:

– either to the action of a compound with the formula $R_1OH$ in which $R_1$ represents the carbonated residue of an alcohol, in order to obtain the corresponding compound with the formula ($I'_B$):

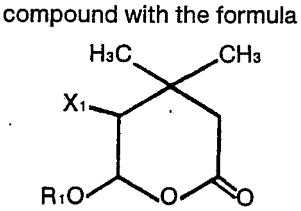

($I'_B$)

in which $R_1$ and $X_1$ are defined as previously;

– or to the action of water in mild conditions in order to obtain the compounds (IV) and ($I'_A$) in equilibrium:

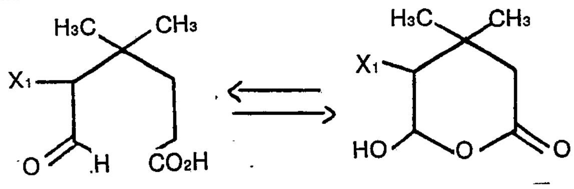

(IV) ($I'_A$)

if desired, the compound with the formula ($I'_A$) is isolated, then if desired, eiher the compound ($I'_A$) or the mixture of compounds (IV) and ($I'_A$), is submitted to the action of an alcohol $R_1OH$ in order to obtain the compound with the formula ($I'_B$):

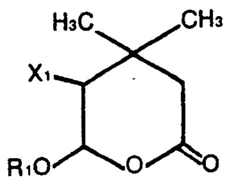

. ($I'_B$)

in which $R_1$ and $X_1$ are defined as previously;

– or to the action of water in more severe conditions, in order to obtain the compound with the formula (V):

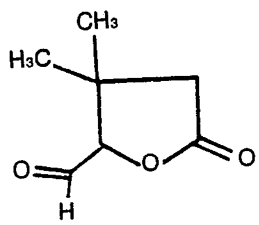

(V)

which is submitted to the action of an alcohol $R_1OH$ in order to obtain the compound with the formula (VI):

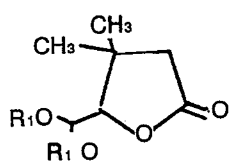

(VI)

which is submitted to the action of a boron trihalogenide:

$(X_1)_3B$

in which $X_1$ is defined as previously, in order to obtain the compound with the formula ($I'_B$):

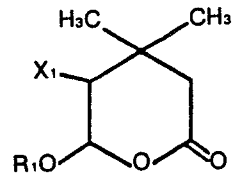

. ($I'_B$)

in which $R_1$ and $X_1$ are defined as previously.

9. As new industrial products, the compounds with the formula (V) and (VI):

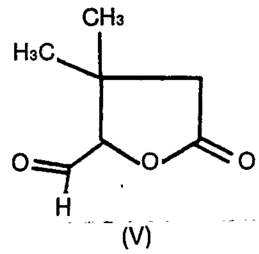

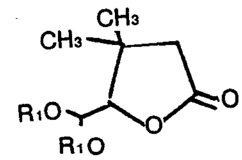

(V) (VI)

in which $R_1$ represents the residue of an alcohol.

10. Application of compounds with the formula (I) as defined in any one of claims 1 to 5, characterized in that a compound with the formula (I):

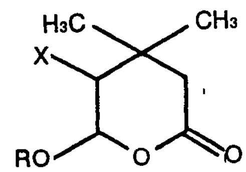

(I)

in which X and R are defined as in claim 1, is submitted to the action of a basic agent in order to obtain the compound with the formula (VII):

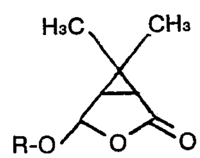

(VII)

in which R is defined as in claim 1.

11. Application according to claim 10, characterized in that the basic agent is an alkali hydroxide.

12. Application according to claim 10 or 11, characterized in that the basic agent is used according to the said method of catalysis by phase transfer.

13. Application according to claim 12, characterized in that in the said method of catalysis by phase transfer, the solvent is methylene chlorid, the catalyst is triethylbenzylammonium, the basic agent is sodium hydroxide and the operation is carried out in the presence of water.